# EUROPEAN PATENT APPLICATION

(11) **EP 2 377 888 A1**
(43) Date of publication of application: **19.10.2011**
(21) Application number: 10003744.9
(22) Date of filing: 07.04.2010
(51) Int. Cl.: C07K 16/28

(54) **Fusion protein**

(71) Applicant: Corimmun GmbH, 82152 Martinsried (DE)
(72) Inventor: Gawaz, Meinrad, 72076 Tübingen (DE); Schönberger, Tanja, 72119 Ammerbuch (DE); Degen, Heidrun, 82110 Germering (DE); Münch, Götz, 80798 München (DE); Holthoff, Hans-Peter, 82377 Penzberg (DE); Bültmann, Andreas, 82152 Planegg (DE); Bühring, Hans-Jörg, 72076 Tübingen (DE); Leder, Christoph, 72076 Tübingen (DE)
(74) Representative: Hartz, Nikolai

(57) **Abstract**

An isolated nucleic acid molecule selected from the group consisting of:
vi. a nucleic acid molecule comprising a nucleotide sequence which is at least 85% identical to the nucleotide sequence of SEQ ID NO:1 or a complement thereof;
vii. a nucleic acid molecule comprising a fragment of at least 1500 consecutive nucleotides of the nucleotide sequence of SEQ ID NO:1, or a complement thereof;
viii. a nucleic acid molecule which encodes a polypeptide comprising an amino acid sequence at least 85% identical to SEQ ID NO:2;
ix. a nucleic acid molecule which encodes a fragment of a polypeptide comprising the amino acid sequence of SEQ ID NO:2, wherein the fragment comprises at least 500 contiguous amino acids of SEQ ID NO: 2; and
x. a nucleic acid molecule encoding a polypeptide containing a humanized immunoglobulin or parts of an immunoglubulin having binding specificity for CD133

a nucleic acid molecule which encodes a variant of a polypeptide comprising the amino acid sequence of SEQ ID NO:2, wherein the nucleic acid molecule hybridizes to a nucleic acid molecule comprising the entire SEQ ID NO: 1, or complement thereof under conditions of incubation at 45 °C in 6.0xSSC followed by washing in 0.2xSSC/0.1 % SDS at 65 °C.

SEQUENCE LISTING

<110>  Corimmun GmbH

<120>  Fusion Protein

<130>  EAD-15297

<160>  6

<170>  PatentIn version 3.1

<210>  1
<211>  2223
<212>  DNA
<213>  Artificial Sequence

<220>
<223>  fusion protein coding sequence

<400>  1
atggaaaccc ctgctcagct gctgttcctg ctgctgctgt ggctgcctga caccaccggc      60

gacatcctga tgacccagtc ccccaagtcc atgtccatgt ccctgggcga gagagtgacc     120

ctgtcctgca aggcctccga gaacgtggac acctacgtgt cctggtatca gcagaagcct     180

gagcagtccc ctaaggtgct gatctacggc gcctccaaca gatacaccgg cgtgcccgac     240

agattcaccg gctccggctc cgccaccgac ttctccctga ccatctccaa cgtgcaggcc     300

gaggacctgg ccgattacca ctgcggccag tcctacagat accctctgac cttcggcgct     360

ggcacaaagc tggaactgaa gggcggaggc ggaagtggag gcggaggatc tggcggcgga     420

ggctctgaag tgcagctgca gcagtccggc cctgacctga tgaagcctgg cgcctccgtg     480

aagatctctt gcaaggccag cggctactcc ttcaccaact actacgtgca ctgggtgaaa     540

cagtccctgg acaagtccct ggaatggatc ggctacgtgg accctttcaa cggcgacttc      600

aactacaacc agaagttcaa ggacaaggcc accctgaccg tggacaagtc tagctccacc     660

gcctacatgc acctgtcctc cctgacctcc gaggactccg ccgtgtacta ctgtgccaga     720

ggcggcctgg attggtacga cacctcctac tggtacttcg acgtgtgggg cgctggaacc     780

gctgtgaccg tgtcctccca gtctggccct ctgcctaagc cttccctgca ggccctgcct     840

tcctccctgg tgcctctgga aaagccagtg accctgcggt gtcagggacc tcctggcgtg     900

gacctgtacc ggctggaaaa gctgtcctcc agcagatacc aggaccaggc cgtgctgttc     960

atccctgcca tgaagcggtc cctggccggc aggtacaggt gctcctacca gaacggctcc    1020

ctgtggtctc tgccttccga ccagctggaa ctggtcgcca caggcgtgtt cgccaagcct    1080

tctctgtctg cccagcctgg ccctgctgtg tcctctggcg gcgacgtgac cctgcagtgc    1140

cagaccagat acggcttcga ccagttcgcc ctgtacaaag agggcgaccc agccccttac    1200

aagaaccctg agcggtggta cagggcctcc ttccctatca tcaccgtgac cgccgctcac    1260

tccggaacct accggtgcta cagcttctcc tcccgggacc cttacctgtg gtccgcccct    1320

agcgaccctc tggaactggt ggtcaccggc acctccgtga ccccttccag gctgcctacc 1380

gagcctccta gctccgtggc cgagttctct gaggccaccg ccgagctgac cgtgtctttc 1440

accaacaagg tgttcaccac cgagacatcc cggtccatca ccacctcccc caaagagtcc 1500

gactctcctg ccggccctgc tcggcagtac tacaccaagg gcaacggcgg cagagtggag 1560

tgtcctcctt gccctgcccc tcctgtggct ggcccttccg tgttcctgtt ccctccaaag 1620

cctaaggaca ccctgatgat ctcccggacc cctgaagtga cctgcgtggt ggtggacgtg 1680

tcccacgagg accctgaggt gcagttcaat tggtacgtgg acggcgtgga ggtgcacaac 1740

gccaagacca agcctcggga ggaacagttc aactccacct tccgggtggt ctctgtgctg 1800

accgtggtgc accaggactg gctgaacggc aaagaataca agtgcaaggt gtccaacaag 1860

ggcctgcctg cccctatcga aaagaccatc agcaagacca agggacagcc tcgcgagcct 1920

caggtgtaca ccctgccacc cagccgggag gaaatgacca agaaccaggt gtccctgacc 1980

tgcctggtca agggcttcta cccttccgat atcgccgtgg agtgggagtc taacggccag 2040

cctgagaaca actacaagac cacccctcct atgctggact ccgacggctc cttcttcctg 2100

tactccaaac tgacagtgga taagtcccgg tggcagcagg gcaacgtgtt ctcctgctct 2160

gtgatgcacg aggccctgca caaccactat acccagaagt ccctgtccct gtctcccggc 2220

aag 2223

<210> 2
<211> 741
<212> PRT
<213> Artificial Sequence

<220>
<223> fusion protein

<400> 2

<210>  3
<211>  356
<212>  DNA
<213>  Artificial Sequence

<220>
<223>  fusion protein coding sequence fragment

<400>  3
gatatcttga tgacccaatc tcccaaatcc atgtccatgt cacttggaga gagggtcacc      60

ttgagctgca aggccagtga gaatgtggat acttatgtat cctggtatca acagaaacca     120

gagcagtctc ctaaagtgtt gatatacggg gcatccaacc ggtacactgg ggtccccgat     180

cgcttcacag gcagtggatc tgcaacagat ttctctctga ccatcagcaa tgtacaggct     240

gaagaccttg cagattatca ctgtggacag agttacaggt atcccctcac gttcggtgct     300

gggaccaagt tggagctgaa acgggctgat gctgcaccaa ctggatccat cttccc         356

<210>  4
<211>  413

<212>  DNA
<213>  Artificial Sequence

<220>
<223>  fusion protein coding sequence fragment

<400>  4
gaggtccagc tgcagcagtc tggacctgac ctgatgaagc ctggggcttc agtgaagata      60

tcctgcaagg cttctggtta ctcattcact aactactacg tgcactgggt gaagcagagc     120

cttgacaaga gccttgagtg gattggatat gttgatcctt tcaatggtga ttttaactac     180

aaccagaaat tcaaggacaa ggccacattg actgtagaca aatcttccag cacagcctac     240

atgcatctca gcagcctgac atctgaggac tctgcagtct attactgtgc cagaggggga     300

cttgactggt atgatacctc ctactggtac ttcgatgtct ggggcgcagg gaccgcggtc     360

accgtctcct cagccaaaac gacacccaag cttgtctatc cactggcccc tgg            413


<210>  5
<211>  873
<212>  DNA
<213>  Artificial Sequence

<220>
<223>  fusion protein coding sequence fragment

<400>  5
atggaaaccc cagcgcagct tctcttcctc ctgctactct ggctcccaga taccaccgga      60

gatatcctga tgacccaatc tcccaaatcc atgtccatgt cacttggaga gagggtcacc     120

ttgagctgca aggccagtga gaatgtggat acttatgtat cctggtatca acagaaacca     180

gagcagtctc ctaaagtgtt gatatacggg gcatccaacc ggtacactgg ggtccccgat     240

cgcttcacag gcagtggatc tgcaacagat ttctctctga ccatcagcaa tgtacaggct     300

gaagaccttg cagattatca ctgtggacag agttacaggt atcccctcac gttcggtgct     360

gggaccaagt tggagctgaa aggtggaggc ggttcaggcg gaggtggcag cggcggtggc     420

ggatcggagg tccagctgca gcagtctgga cctgacctga tgaagcctgg ggcttcagtg     480

aagatatcct gcaaggcttc tggttactca ttcactaact actacgtgca ctgggtgaag     540

cagagccttg acaagagcct tgagtggatt ggatatgttg atcctttcaa tggtgatttt     600

aactacaacc agaaattcaa ggacaaggcc acattgactg tagacaaatc ttccagcaca     660

gcctacatgc atctcagcag cctgacatct gaggactctg cagtctatta ctgtgccaga     720

gggggacttg actggtatga tacctcctac tggtacttcg atgtctgggg cgcagggacc     780

gcggtcaccg tctcctcagc caaaacgaca cccaagcttg tctatccact ggcccctgga     840

tccgcttggt cccacccgca gttcgagaaa taa                                  873


<210>  6
<211>  864

<212>  DNA
<213>  Artificial Sequence

<220>
<223>  fusion protein coding sequence fragment

<400>  6
atgaaacacc tgtggttctt cctcctgctg gtggcagctc ccagatgggt cctgtccgag      60

gtccagctgc agcagtctgg acctgacctg atgaagcctg gggcttcagt gaagatatcc     120

tgcaaggctt ctggttactc attcactaac tactacgtgc actgggtgaa gcagagcctt     180

gacaagagcc ttgagtggat tggatatgtt gatcctttca atggtgattt taactacaac     240

cagaaattca aggacaaggc cacattgact gtagacaaat cttccagcac agcctacatg     300

catctcagca gcctgacatc tgaggactct gcagtctatt actgtgccag agggggactt     360

gactggtatg atacctccta ctggtacttc gatgtctggg gcgcagggac cgcggtcacc     420

gtctcctcag gtggaggcgg ttcaggcgga ggtggcagcg gcggtggcgg atcggatatc     480

ctgatgaccc aatctcccaa atccatgtcc atgtcacttg gagagagggt caccttgagc     540

tgcaaggcca gtgagaatgt ggatacttat gtatcctggt atcaacagaa accagagcag     600

tctcctaaag tgttgatata cggggcatcc aaccggtaca ctggggtccc cgatcgcttc     660

acaggcagtg gatctgcaac agatttctct ctgaccatca gcaatgtaca ggctgaagac     720

cttgcagatt atcactgtgg acagagttac aggtatcccc tcacgttcgg tgctgggacc     780

aagttggagc tgaaacgggc tgatgctgca ccaactggat ccatcttccc atccgcttgg     840

tcccacccgc agttcgagaa ataa                                            864

## Description

### FIELD OF THE INVENTION

The present invention relates to a nucleic acid molecule and a polypeptide capable of simultaneously and selectively binding to collagen and CD133 protein. The present invention also relates to a host cell which contains the nucleic acid molecule of the invention. Moreover, the present invention relates to a method for producing the polypeptide of the invention. The polypeptide may be used for the prevention, treatment or diagnosis of cardiovascular disease. Accordingly, the present invention also relates to a pharmaceutical composition containing the polypeptide of the invention, which is preferably a dimer.

### BACKGROUND OF THE INVENTION

Endothelial progenitor cells (EPC) reside in the bone marrow and are released into the blood stream, where they are involved in hemostasis and tissue repair. CD133 protein, a pentaspan transmembrane glycoprotein, is expressed on the surface of EPCs whereby expression is down-regulated upon differentiation of the EPCs into endothelial cells. CD133 is not expressed on any other cell type of the blood, which makes it an attractive target for the recruitment of EPCs.

A bispecific protein which is able to attract endothelial progenitor cells (EPC) to sites of vascular lesions is known from WO 2008/101700. The protein disclosed by WO 2008/101700 contains a moiety capable of binding to CD133 on EPCs with high affinity. Moreover, the protein disclosed by WO 2008/101700 contains a moiety capable of recognizing and binding to lesions in the endothelial lining of blood vessels. According to WO 2008/101700 the protein is prepared by linking a first protein capable of binding to endothelial precursor cells and a second protein capable of binding collagen.

The first and second proteins are linked by using SPDP (N-succinimidyl 3-(2-pyridyldithio)-propionate), which is a heterobifunctional crosslinking agent. Since the first protein contains about 25 lysine residues reactive with SPDP, and the second protein contains about 18 lysine residues, the number of possible dimeric products is at least 450 (18 × 25). Additionally, the formation of higher oligomers cannot be avoided. Accordingly, the crosslinked product contains a heterogenous mixture of products. Incidentally, the mixture does not contain any fusion protein of the first and second proteins since the products are not prepared through the joining of two or more genes which code for the first and second proteins.

Accordingly, none of the products made available by WO 2008/101700 may be considered to be the product of a translation of a fusion gene or as a single polypeptide with functional properties derived from each of the original proteins.

The protein mixture disclosed by WO 2008/101700 is unsuitable for use as a medicament since the mixture cannot be provided with a standardized and defined composition and sufficient purity for it to be suitable for therapeutic application. Moreover, since oligomers cannot be avoided, the yield and efficacy of the mixture of WO 2008/101700 is problematic.

On the other hand, attempts to prepare fusion protein containing polypeptide sequences present in the mixture of WO 2008/101700 were unable to bind a second protein capable of selectively and simultaneously binding to endothelial precursor cells and collagen.

### SUMMARY OF THE INVENTION

Therefore, it is the aim of the present invention to provide a polypeptide of a small size which is capable of simultaneously and selectively binding to collagen and CD133 protein, whereby the protein may be prepared in high yield and high purity to be useful in a pharmaceutical composition for augmenting healing processes directly by differentiation of EPCs into endothelial cells of the vessel wall or indirectly by secretion of positive modulating factors.

The present invention provides nucleic acid molecules encoding a specific fusion protein. The fusion protein may be used in the treatment or prevention of cardiovascular disease by homing EPCs to exposed collagen or for diagnostic purposes.

According to a first aspect the present invention provides an isolated nucleic acid molecule selected from the group consisting of:
i. a nucleic acid molecule comprising a nucleotide sequence which is at least 85% identical to the nucleotide sequence of SEQ ID NO:1 or a complement thereof;
ii. a nucleic acid molecule comprising a fragment of at least 1500 consecutive nucleotides of the nucleotide sequence of SEQ ID NO:1, or a complement thereof;
iii. a nucleic acid molecule which encodes a polypeptide comprising an amino acid sequence at least 85% identical to SEQ ID NO:2;
iv. a nucleic acid molecule which encodes a fragment of a polypeptide comprising the amino acid sequence of SEQ ID NO:2, wherein the fragment comprises at least 500 contiguous amino acids of SEQ ID NO: 2; and
v. a nucleic acid molecule which encodes a variant of a polypeptide comprising the amino acid sequence of SEQ ID NO: 2, wherein the nucleic acid molecule hybridizes to a nucleic acid molecule comprising the entire SEQ ID NO: 1, or complement thereof under conditions of incubation at 45 °C in 6.0xSSC followed by washing in 0.2xSSC/0.1% SDS at 65 °C.

The nucleic acid sequence of SEQ ID NO:1 is as follows:

ATGGAAACCCCTGCTCAGCTGCTGTTCCTGCTGCTGCTGTGGCTGCCTGACAC
CACCGGCGACATCCTGATGACCCAGTCCCCCAAGTCCATGTCCATGTCCCTGG
GCGAGAGAGTGACCCTGTCCTGCAAGGCCTCCGAGAACGTGGACACCTACGT
GTCCTGGTATCAGCAGAAGCCTGAGCAGTCCCCTAAGGTGCTGATCTACGGC
GCCTCCAACAGATACACCGGCGTGCCCGACAGATTCACCGGCTCCGGCTCCG
CCACCGACTTCTCCCTGACCATCTCCAACGTGCAGGCCGAGGACCTGGCCGA
TTACCACTGCGGCCAGTCCTACAGATACCCTCTGACCTTCGGCGCTGGCACAA
AGCTGGAACTGAAGGGCGGAGGCGGAAGTGGAGGCGGAGGATCTGGCGGCG
GAGGCTCTGAAGTGCAGCTGCAGCAGTCCGGCCCTGACCTGATGAAGCCTGG
CGCCTCCGTGAAGATCTCTTGCAAGGCCAGCGGCTACTCCTTCACCAACTACT
ACGTGCACTGGGTGAAACAGTCCCTGGACAAGTCCCTGGAATGGATCGGCTA

CGTGGACCCTTTCAACGGCGACTTCAACTACAACCAGAAGTTCAAGGACAAGG
CCACCCTGACCGTGGACAAGTCTAGCTCCACCGCCTACATGCACCTGTCCTCC
CTGACCTCCGAGGACTCCGCCGTGTACTACTGTGCCAGAGGCGGCCTGgATT
GGTACGACACCTCCTACTGGTACTTCGACGTGTGGGGCGCTGGAACCGCTGT
GACCGTGTCCTCCCAGTCTGGCCCTCTGCCTAAGCCTTCCCTGCAGGCCCTG
CCTTCCTCCCTGGTGCCTCTGGAAAAGCCAGTGACCCTGCGGTGTCAGGGAC
CTCCTGGCGTGGACCTGTACCGGCTGGAAAAGCTGTCCTCCAGCAGATACCA
GGACCAGGCCGTGCTGTTCATCCCTGCCATGAAGCGGTCCCTGGCCGGCAGG
TACAGGTGCTCCTACCAGAACGGCTCCCTGTGGTCTCTGCCTTCCGACCAGCT
GGAACTGGTCGCCACAGGCGTGTTCGCCAAGCCTTCTCTGTCTGCCCAGCCT
GGCCCTGCTGTGTCCTCTGGCGGCGACGTGACCCTGCAGTGCCAGACCAGAT
ACGGCTTCGACCAGTTCGCCCTGTACAAAGAGGGCGACCCAGCCCCTTACAA
GAACCCTGAGCGGTGGTACAGGGCCTCCTTCCCTATCATCACCGTGACCGCC
GCTCACTCCGGAACCTACCGGTGCTACAGCTTCTCCTCCCGGGACCCTTACCT
GTGGTCCGCCCCTAGCGACCCTCTGGAACTGGTGGTCACCGGCACCTCCGTG
ACCCCTTCCAGGCTGCCTACCGAGCCTCCTAGCTCCGTGGCCGAGTTCTCTGA
GGCCACCGCCGAGCTGACCGTGTCTTTCACCAACAAGGTGTTCACCACCGAG
ACATCCCGGTCCATCACCACCTCCCCCAAAGAGTCCGACTCTCCTGCCGGCCC
TGCTCGGCAGTACTACACCAAGGGCAACGGCGGCAGAGTGGAGTGTCCTCCT
TGCCCTGCCCCTCCTGTGGCTGGCCCTTCCGTGTTCCTGTTCCCTCCAAAGCC
TAAGGACACCCTGATGATCTCCCGGACCCCTGAAGTGACCTGCGTGGTGGTG
GACGTGTCCCACGAGGACCCTGAGGTGCAGTTCAATTGGTACGTGGACGGCG
TGGAGGTGCACAACGCCAAGACCAAGCCTCGGGAGGAACAGTTCAACTCCAC
CTTCCGGGTGGTCTCTGTGCTGACCGTGGTGCACCAGGACTGGCTGAACGGC
AAAGAATACAAGTGCAAGGTGTCCAACAAGGGCCTGCCTGCCCCTATCGAAAA
GACCATCAGCAAGACCAAGGGACAGCCTCGCGAGCCTCAGGTGTACACCCTG
CCACCCAGCCGGGAGGAAATGACCAAGAACCAGGTGTCCCTGACCTGCCTGG
TCAAGGGCTTCTACCCTTCCGATATCGCCGTGGAGTGGGAGTCTAACGGCCAG
CCTGAGAACAACTACAAGACCACCCCTCCTATGCTGGACTCCGACGGCTCCTT
CTTCCTGTACTCCAAACTGACAGTGGATAAGTCCCGGTGGCAGCAGGGCAACG
TGTTCTCCTGCTCTGTGATGCACGAGGCCCTGCACAACCACTATACCCAGAAG
TCCCTGTCCCTGTCTCCCGGCAAG

According to a second aspect, the present invention provides a host cell which contains the nucleic acid molecule of the first aspect of the present invention.

According to a third aspect, the present invention provides a polypeptide capable of simultaneously and selectively binding to collagen and CD133 protein, which is selected from the group consisting of:
a) a fragment of a polypeptide comprising the amino acid sequence of SEQ ID NO: 2, wherein the fragment comprises at least 600 contiguous amino acids of SEQ ID NO: 2;
b) a variant of a polypeptide comprising the amino acid sequence of SEQ ID NO: 2, wherein the variant is encoded by a nucleic acid molecule which hybridizes to a nucleic acid molecule comprising the entire SEQ ID NO:1 or a complement thereof under conditions of incubation at 45 °C in 6.0xSSC followed by washing in 0.2xSSC/0.1% SDS at 65 °C.;
c) a polypeptide which is encoded by a nucleic acid molecule comprising a nucleotide sequence which is at least 85% identical to a nucleic acid consisting of the nucleotide sequence of SEQ ID NO:1 or the complement thereof; and
d) a polypeptide comprising an amino acid sequence that is at least 85% identical to SEQ ID NO:2.

The polypeptide according to the third aspect is advantageous for use in the prevention or treatment of cardiovascular disease.

The amino acid sequence of SEQ ID NO:2 is as follows:

According to a fourth aspect, the present invention provides a method for producing a polypeptide comprising the amino acid sequence of SEQ ID NO: 2, the method comprising culturing the host cell according to the third aspect under conditions in which the nucleic acid molecule is expressed.

According to the fifth aspect, the present invention provides a pharmaceutical composition comprising the polypeptide according to the third aspect of the invention.

According to a sixth aspect, the present invention provides a use of a polypeptide according to the third aspect for the manufacture of a medicament for the prevention or treatment of cardiovascular disease.

The present invention demonstrates that a fusion protein according to the present invention which may be expressed in mammalian cell culture with high efficiency, is capable of binding its targets CD133 and collagen with high affinity, and of immobilizing CD133 expressing HEK 293 cells to a collagen coated surface even under dynamic conditions as shown in the flow chamber experiment.

Comparable to the protein mixture of WO 2008/101700 which contains W6B3H10 mAb chemically linked to GPVI-Fc, the fusion protein of the present invention is able to recruit CD133 positive progenitor cells, isolated from human cord blood, to induced vascular lesions in a mouse model. Moreover, the recruited EPCs differentiate to endothelial cells and thus directly contributed to regeneration of the endothelial wall. Therefore, the fusion protein of the present invention augments reendothelialization and is of beneficial value in regenerative vascular medicine. A polypeptide of the invention is useful for augmenting healing processes directly by differentiation into endothelial cells of the vessel wall or indirectly by secretion of positive modulating factors.

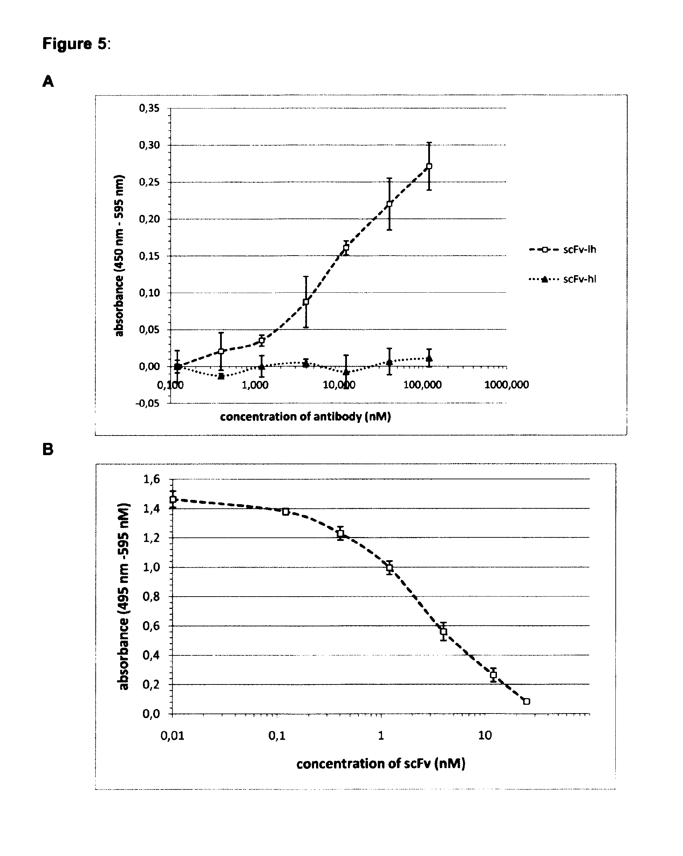

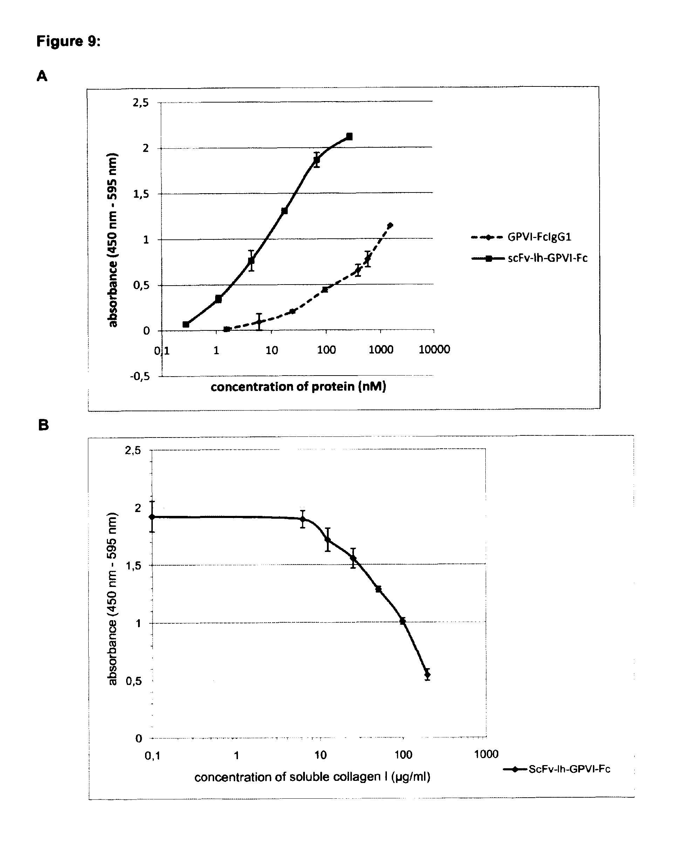

Superior to the existing chemically linked constructs, the novel fusion protein has higher affinities to collagen (see figure 9). This presents an advantage for the use as a medicament with higher efficacy for the local binding to vascular lesions. Of the various possibilities of the fusion protein, only scFv-Ih showed comparable high affinity to CD133, whereas other derived constructs did not (see figure 5).

Beside the application of the polypeptide of the present invention for vascular regeneration processes, it can also be employed to improve homing of transplanted stem cells to the bone marrow after bone marrow ablation by chemotherapy or radiotherapy.

The present invention provides a polypeptide according to SEQ ID NO: 2 of a small size which is capable of simultaneously and selectively binding to collagen and CD133 protein. The protein according to the present invention may be prepared in high yield and high purity, which makes it highly useful in a pharmaceutical composition.

The polypeptide according to the invention is a fusion protein, i.e. the product of a translation of a fusion gene. The fusion protein contains a domain of a single chain anti-CD133 antibody, a linker, an Fc portion, and a GPVI portion. The protein of the present invention may be in the form of a dimer.

The polypeptide according to the present invention is based on a single chain antibody (scFv), which is composed solely of the variable sequences of the light and heavy chains of a monoclonal antibody, which are combined on one polypeptide chain by a connecting linker peptide. These single chain antibodies retain the specificity to the antigen of the parental mAb with surprisingly high affinity. The parental mAb used according to the present invention may be produced by the mouse hybridoma cell clone W6B3H10, a subclone of the commercially available clone W6B3C1 (Miltenyi, Bergisch Gladbach).

The second fusion partner is capable of recognizing and binding to lesions in the endothelial lining of blood vessels. After injury of the endothelial cell layer by surgical intervention such as stent implantation or after rupture of atherosclerotic plaques, collagen, a constituent of the subendothelial matrix, is exposed to the blood stream. This leads to rapid attachment and activation of platelets, which in turn can cause thrombus formation and finally occlusion of the blood vessel.

Platelets adhere to collagen via glycoprotein VI (GPVI), a membrane glycoprotein receptor, which is expressed on the surface of platelets. The soluble portion of human platelet glycoprotein VI, which corresponds to the extracellular domain of the protein, shows high binding affinity to collagen.

GPVI binds collagen with high affinity as a homodimer. To facilitate dimerization on the one hand and purification of the fusion protein by affinity chromatography on the other, the Fc portion of human IgG is attached to soluble GPVI portion. The Fc-fragment forms dimers via covalent disulfide bonds in the remaining part of the hinge region, which promotes dimerization of GPVI probably supported by disulfide bond formation. Moreover, the Fc-tag increases the half-life of the fusion protein in the blood stream.

### BRIEF DESCRIPTION OF THE DRAWINGS

Figure 1 shows assembled sequences of the W6B3H10 light and heavy variable region cDNAs. Specifically, figure 1A shows the sequence of a kappa light chain. The underlined sequence belongs to the constant region of the light chain sequence. Figure 1B shows a sequence for a gamma heavy chain.
Figure 2 shows the nucleotide sequence of the constructs scFv-Ih depicted in A and scFv-hI shown in B. The underlined sequence in A is derived from the constant region of the heavy chain, in B from the constant region of the light chain. The Gly-Ser linker sequence is written in italics.
Figure 3 shows a Western blot of the purification of the single chain antibody scFv-Ih from CHO cell supernatant using Strep-Tactin matrix, detected with StrepMAb-Classic-HRP antibody. The flow through (FT), the first two wash fractions (W1, W2), eluate fractions 1 to 5 (E1-E5) and the matrix after elution (M) is shown. The specific band is shown at the expected size of ca. 27 kDalton in lanes E2-E5. Lane M shows non-specific signals.
Figure 4 shows a Coomassie gel of the purification of scFv-hl from bacteria. B, bacterial lysate, E2/3, combined eluates 2 and 3, E4, E5, eluate fraction 4 and 5.
Figure 5 shows binding of the single chain antibodies to CD133 on fixed AC133/293 cells. A, concentration dependent binding properties B, competition of binding of 2 nmol/L W6B3H10 mAb to CD133 on fixed AC133/293 cells by the single chain antibody scFv-lh.
Figure 6 shows nucleotide sequence (SEQ ID NO 1) and amino acid sequence (SEQ ID NO: 2) of the fusion protein scFv-lh-GPVI-Fc. Figure 1A shows the nucleotide sequence which is codon-optimized for efficient expression in CHO cells, whereby the sequence coding for the single chain moiety is underlined. Figue 1 B shows the amino acid sequence (SEQ ID NO 2) which is deduced from the nucleotide sequence. The 20 amino acid leader peptide shown is absent in the mature protein. The sequence of the single chain moiety is underlined. GPVI and FclgG2 are connected by a GGR-linker shown in bold.
Figure 7 shows the fusion protein which was separated on a 4-20% polyacrylamide gel under non-reducing and reducing conditions, the gel was stained with Coomassie Brilliant Blue.
Figure 8 shows the characterization of binding of the fusion protein to CD133 on fixed AC133/293 cells. Figure 8A shows titration ELISA for comparison of binding of the fusion protein and the parental mAb W6B3H10. Figure 8B shows competitive ELISA with 2 nM W6B3H10 mAb and scFv-lh-GPVI-Fc as competitor.
Figure 9 shows measurement of binding of the fusion protein compared to GPVI-FclgG1 to 0.1 µg bovine collagen I by ELISA. Figure 9A demonstrates concentration dependent binding. Figure 9B demonstrates competition of binding of the fusion protein to 1 µg/ml immobilized collagen I, competed by increasing amounts of soluble collagen I.
Figure 10 demonstrates fusion protein mediated binding of CD133-expressing cells and of HEK 293 control cells to collagen under shear forces of 2000/s.
Figure 11 demonstrates fusion protein mediated binding of qEPCs to the carotid artery of mouse after ligation induced injury in vivo, measured by intravital fluorescence microscopy.

### DESCRIPTION OF THE PREFERRED EMBODIMENTS

A a polypeptide is capable of simultaneously binding to collagen and CD133 protein when the polypeptide may exist in a state where it forms a bridge between a CD133 protein and a collagen protein, in particular under the conditions described in the present examples. Amino acid or nucleotide sequences having about 85% identity, preferably 90%, 95%, or 98% identity with SEQ ID NO: 1 or SEQ ID NO: 2, respectively, are defined herein as sufficiently identical. Accordingly, the term " sufficiently identical" refers to a first amino acid or nucleotide sequence which contains a sufficient or minimum number of identical or equivalent (e.g., an amino acid residue which has a similar side chain) amino acid residues or nucleotides to the second amino acid or nucleotide sequence (SEQ ID NO: 1 or SEQ ID NO: 2) such that the first and second amino acid or nucleotide sequences have a common structural domain and/or common functional activity. As used herein, a "fusion protein" is a polypeptide exerting fusion protein activity. As used herein a "fusion protein activity", "biological activity of fusion protein " or "functional activity of fusion protein" refers to the simultaneous and selective binding to collagen and CD133 protein.

### Nucleic acid molecules

The invention features a nucleic acid molecule which is sufficiently identical by being at least 85% (90%, 95%, or 98%) identical to the nucleotide sequence shown in SEQ ID NO: 1, or a complement thereof.

The present invention features a nucleic acid molecule which includes a fragment of at least 1500 (1600, 1800, 2000, 2200) nucleotides of the nucleotide sequence shown in SEQ ID NO: 1, or a complement thereof. In an embodiment, a nucleic acid molecule according to the present invention has the nucleotide sequence shown in SEQ ID NO: 1. Also within the invention is a nucleic acid molecule which encodes a fragment of a polypeptide having the amino acid sequence of SEQ ID NO: 2.

The invention also includes a nucleic acid molecule encoding a polypeptide, wherein the nucleic acid hybridizes to a nucleic acid molecule consisting of SEQ ID NO: 2 under stringent conditions (e.g., hybridization in 6*sodium chloride/sodium citrate (SSC) at about 60 °C, followed by one or more washes in 0.2*SSC, 0.1 % SDS at 65°C.), and wherein the nucleic acid encodes a polypeptide of at least 500 amino acids in length, preferably at least 700 amino acids, having a molecular weight of approximately 65 to 85 kD prior to post-translational modifications and in reduced form.

One aspect of the invention pertains to isolated nucleic acid molecules that encode fusion proteins or biologically active portions thereof, as well as nucleic acid molecules sufficient for use as hybridization probes to identify fusion protein - encoding nucleic acids (e.g., fusion protein mRNA) and fragments for use as PCR primers for the amplification or mutation of fusion protein nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid. Preferably, an "isolated" nucleic acid is free of sequences (preferably protein encoding sequences) which naturally flank the nucleic acid in the genomic DNA of the organism from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, e.g., a nucleic acid molecule having the nucleotide sequence of SEQ ID NO: 1, or a complement of any of this nucleotide sequences, can be isolated using standard molecular biology techniques and the sequence information provided herein (Sambrook et al., Molecular Cloning: A Laboratory Manual. 2nd, ed., Cold Spring Harbor Laboratory, Cold Spring Harbor Laboratory Press, Cold Spring Harbor, N.Y., 1989).

In another embodiment, an isolated nucleic acid molecule of the invention comprises a nucleic acid molecule which is a complement of the nucleotide sequence shown in SEQ ID NO: 1, or a portion thereof. A nucleic acid molecule which is complementary to a given nucleotide sequence is one which is sufficiently complementary to the given nucleotide sequence that it can hybridize to the nucleotide sequence thereby forming a stable duplex.

Moreover, the nucleic acid molecule of the invention can comprise only a portion of a nucleic acid sequence encoding fusion protein, e.g. a fragment which can be used as a probe or primer or a fragment encoding a biologically active portion of fusion protein.

A nucleic acid fragment encoding a "biologically active portion" of fusion protein can be prepared by isolating a portion of SEQ ID NO: 1, which encodes a polypeptide having a fusion protein biological activity, expressing the encoded portion of fusion protein (e.g., by recombinant expression *in vitro*) and assessing the activity of the encoded portion of fusion protein.

The invention further encompasses nucleic acid molecules that differ from the nucleotide sequence of SEQ ID NO: 1 due to degeneracy of the genetic code and thus encode the same fusion protein as that encoded by the nucleotide sequence shown in SEQ ID NO: 1.

Accordingly, in another embodiment, an isolated nucleic acid molecule of the invention is at least 1500 (1600, 1800, 2000, 2200) nucleotides in length and hybridizes under stringent conditions to the nucleic acid molecule comprising the nucleotide sequence, preferably the coding sequence, of SEQ ID NO: 1.

As used herein, the term "hybridizes under stringent conditions" is intended to describe conditions for hybridization and washing under which nucleotide sequences at least 85% (95%, 98%) identical to each other typically remain hybridized to each other. Such stringent conditions are known to those skilled in the art and can be found in Current Protocols in Molecular Biology, John Wiley & Sons, N.Y. (1989), 6.3.1-6.3.6. An example of stringent hybridization conditions are hybridization in 6*sodium chloride/sodium citrate (SSC) at about 4°C., followed by one or more washes in 0.2*SSC, 0.1 % SDS at 50-65 °C (e.g., 50 °C. or 60 °C. or 65 °C.). Preferably, the isolated nucleic acid molecule of the invention that hybridizes under stringent conditions corresponds to a naturally-occurring nucleic acid molecule.

Changes can be introduced by mutation into the nucleotide sequence of SEQ ID NO: 1, thereby leading to changes in the amino acid sequence of the encoded protein without altering the functional ability of the fusion protein. For example, nucleotide substitutions may be made which lead to amino acid substitutions at "non-essential" amino acid residues. A "non-essential" amino acid residue is a residue that can be altered from the sequence of SEQ ID NO: 2 without altering the biological activity, whereas an "essential" amino acid residue is required for biological activity of the fusion protein.

Accordingly, another aspect of the invention pertains to nucleic acid molecules encoding fusion proteins that contain changes in amino acid residues that are not essential for activity. Such fusion proteins differ in amino acid sequence from SEQ ID NO: 2 and yet retain biological activity.

In one embodiment, the nucleic acid molecule includes a nucleotide sequence encoding a protein that includes an amino acid sequence that is at least about 85%, 95%, or 98% identical to the amino acid sequence of SEQ ID NO: 2. An isolated nucleic acid molecule encoding a fusion protein having a sequence which differs from that of SEQ ID NO: 1, can be created by introducing one or more nucleotide substitutions, additions or deletions into the nucleotide sequence of fusion protein (SEQ ID NO: 1) such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. Thus, for example, 1%, 2%, 3%, 5%, or 10% of the amino acids can be replaced by conservative substitution. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a fusion protein is preferably replaced with another amino acid residue from the same side chain family. Alternatively, mutations can be introduced randomly along all or part of a fusion protein coding sequence, such as by saturation mutagenesis, and the resultant mutants can be screened for fusion protein biological activity to identify mutants that retain activity. Following mutagenesis, the encoded protein can be expressed recombinantly and the activity of the protein can be determined.

A mutant fusion protein can be assayed for the ability to simultaneously and selectively bind to CD133 and collagen.

The invention also relates to isolated nucleic acid molecules comprising a nucleic acid sequence which encodes a humanized immunoglobulin of the present invention (e.g., a single chain antibody), as well as to isolated nucleic acid molecules comprising a sequence which encodes a humanized immunoglobulin light chain (e.g., a sequence encoding an amino acid sequence of SEQ ID NO:12, 13, 14, or 15 and/or a sequence encoding an amino acid of SEQ ID NO: 111, 116 or portions thereof) or heavy chain (e.g., a sequence encoding an amino acid sequence of SEQ ID NO: 17, 18, 19 or 20 and/or a sequence encoding an amino acid sequence of SEQ ID NO: 110, 114 or portions thereof.

The present invention further relates to a nucleic acid molecule encoding a fusion protein containing a humanized immunoglobulin having binding specificity for CD133 or parts of a chain of such an immunoglobulin. For example, an expression vector comprising a gene encoding a humanized immunoglobulin light chain, comprising a nucleotide sequence encoding a CDR derived from a light chain of a nonhuman antibody having binding specificity for CD133, and a framework region derived from a light chain of human origin, is provided. An expression vector comprising a gene encoding a humanized immunoglobulin heavy chain, comprising a nucleotide sequence encoding a CDR derived from a heavy chain of a nonhuman antibody having binding specificity for CD133, and a framework region derived from a heavy chain of human origin is another example of such a construct. In one embodiment, the expression vector can include a nucleic acid encoding a light chain that includes a first nucleic acid sequence encoding a light chain variable region, e.g., from SEQ ID NO: 1 (nt 61 to 381), and a second nucleic acid sequence encoding a heavy chain variable region, e.g. from SEQ ID NO: 1 (nt 427 to 798) or a portion thereof. In some embodiments, the expression vector can include a nucleic acid encoding a light chain as described herein and a nucleic acid encoding a heavy chain as described herein.

### Isolated fusion protein and anti-fusion protein antibodies

The present invention also relates to a polypeptide having an amino acid sequence that is at least 85%, preferably 95% or 98% identical to the amino acid sequence of SEQ ID NO: 2.

One aspect of the invention pertains to isolated fusion proteins, and biologically active portions thereof, as well as polypeptide fragments suitable for use as immunogens to raise anti-fusion protein antibodies. In one embodiment, fusion proteins are produced by recombinant DNA techniques. Alternative to recombinant expression, a fusion protein or polypeptide can be synthesized chemically using standard peptide synthesis techniques.

An "isolated" or "purified" protein or biologically active portion thereof is substantially free of cellular material or other contaminating proteins from the cell or tissue source from which the fusion protein is derived, or substantially free from chemical precursors or other chemicals when chemically synthesized. The term "substantially free of cellular material" includes preparations of fusion protein in which the protein is separated from cellular components of the cells from which it is isolated or recombinantly produced. Thus, fusion protein that is substantially free of cellular material includes preparations of fusion protein having less than about 30%, 20%, 10%, or 5% (by dry weight) of non-fusion protein (also referred to herein as a "contaminating protein"). When the fusion protein or biologically active portion thereof is recombinantly produced, it is also preferably substantially free of culture medium, i.e., culture medium represents less than about 20%, 10%, or 5% of the volume of the protein preparation. When fusion protein is produced by chemical synthesis, it is preferably substantially free of chemical precursors or other chemicals, i.e., it is separated from chemical precursors or other chemicals which are involved in the synthesis of the protein. Accordingly such preparations of fusion protein have less than about 30%, 20%, 10%, 5% (by dry weight) of chemical precursors or non-fusion protein chemicals.

Biologically active portions of a fusion protein include peptides comprising amino acid sequences sufficiently identical to or derived from the amino acid sequence of the fusion protein (e.g., the amino acid sequence shown in SEQ ID NO: 2), which include less amino acids than the full length fusion protein, and exhibit at least a fusion protein activity. Typically, biologically active portions comprise a domain or motif with at least fusion protein activity. A biologically active portion of a fusion protein can be a polypeptide which is, for example, at least 500, 550, 600, 650, or 700 amino acids in length. Preferred biologically active polypeptides include one or more fusion protein structural domains, in particular a domain derived from a single chain antibody selectively binding CD133, a linker, an Fc portion and a GPVI portion.

A useful fusion protein is a protein which includes an amino acid sequence at least about 85%, preferably 95% or 99% identical to the amino acid sequence of SEQ ID NO: 2 and retains the functional activity of the fusion protein of SEQ ID NO: 2.

Because the antibody moiety of the fusion protein is derived from a mouse monoclonal antibody, the fusion protein is a mouse-human chimeric protein, whose mouse sequences except those being involved in antigen recognition, may be replaced against human sequences by antibody humanization. Generally, partially human antibodies and fully human antibodies have a longer half-life within the human body than other antibodies. Accordingly, lower dosages and less frequent administration are often possible. Modifications such as lipidation can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193).

The determination of percent homology between two sequences can be accomplished using a mathematical algorithm. A preferred, non-limiting example of a mathematical algorithm utilized for the comparison of two sequences is the algorithm of Karlin and Altschul (1990) Proc. Nat'l Acad. Sci. USA 87:2264-2268, modified as in Karlin and Altschul (1993) Proc. Nat'l Acad. Sci. USA 90:5873-5877. Such an algorithm is incorporated into the NBLAST and XBLAST programs of Altschul, et al. (1990) J. Mol. Biol. 215:403-410. BLAST nucleotide searches can be performed with the NBLAST program, score=100, wordlength=12 to obtain nucleotide sequences similar or homologous to nucleic acid molecules of the invention. To obtain gapped alignments for comparison purposes, Gapped BLAST can be utilized as described in Altschul et al. (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov.

The percent identity between two sequences can be determined using techniques similar to those described above, with or without allowing gaps. In calculating percent identity, typically exact matches are counted.

Preferably, a fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. An isolated fusion protein, or a portion or fragment thereof, can be used as an immunogen to generate antibodies that bind the fusion protein using standard techniques for polyclonal and monoclonal antibody preparation.

The full-length fusion protein can be used or, alternatively, the invention provides antigenic peptide fragments of fusion protein for use as immunogens. The antigenic peptide of fusion protein comprises at least 8 (preferably 10, 15, 20, or 30) amino acid residues of the amino acid sequence shown in SEQ ID NO: 2, and encompasses an epitope of fusion protein such that an antibody raised against the peptide forms a specific immune complex with fusion protein.

The present invention also provides a polypeptide containing a variable region of a humanized immunoglobulin having binding specificity for CD133. Specifically, the present invention also relates to polypeptide of a fusion protein of the present invention containing a humanized immunoglobulin fragment having binding specificity for CD133, wherein the immunoglobulin comprises an antigen binding region of nonhuman origin (e.g., rodent) and at least a portion of an immunoglobulin of human origin (e.g., a human framework region, a human constant region of the gamma type).

In some embodiments, the polypeptide of the present invention can further include all or a portion of a constant region of human origin, e.g., all or a portion of a human heavy chain constant region and/or a human light chain constant region. Moreover, the polypeptide of the present invention may comprise a humanized immunoglobulin including all or a portion of human constant region having one or more mutations, e.g., one or more mutations that reduce binding to Fc receptors and/or the ability to fix complement.

A fusion protein immunogen may be used to prepare antibodies by immunizing a suitable subject, e.g., rabbit, goat, mouse or other mammal, with the immunogen. Immunization of a suitable subject with an immunogenic fusion protein preparation induces a polyclonal anti-fusion protein antibody response. Accordingly, another aspect of the invention pertains to anti-fusion protein antibodies.

Polyclonal anti-fusion protein antibodies can be prepared by immunizing a suitable subject with a fusion protein immunogen. The antibody molecules directed against fusion protein can be isolated from the mammal (e.g., from the blood) and further purified by well-known techniques, such as protein A chromatography to obtain the IgG fraction. At an appropriate time after immunization, antibody-producing cells can be obtained from the subject and used to prepare monoclonal antibodies by standard techniques, such as the hybridoma technique described by Kohler and Milstein (1975) Nature 256:495-497, the human B cell hybridoma technique (Kozbor et al. (1983) Immunol Today 4:72), or the EBV-hybridoma technique (Cole et al. (1985), Monoclonal Antibodies and Cancer Therapy, Alan R. Liss, Inc., pp. 77-96).

### Recombinant Expression Vectors and Host Cells

Another aspect of the invention pertains to vectors, preferably expression vectors, containing a nucleic acid encoding the fusion protein (or a portion thereof).

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Vectors may be capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. In general, expression vectors of utility in recombinant DNA techniques are preferably in the form of plasmids (vectors). However, the invention also includes such other forms of expression vectors, such as viral vectors (e.g., replication defective retroviruses, adenoviruses and adeno-associated viruses).

The recombinant expression vectors of the invention comprise a nucleic acid of the invention in a form suitable for expression of the nucleic acid in a host cell. Accordingly, the recombinant expression vectors include one or more regulatory sequences, selected on the basis of the host cells to be used for expression, which is operatively linked to the nucleic acid sequence to be expressed.

Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell).

The term "regulatory sequence" is intended to include promoters, enhancers and other expression control elements (e.g., polyadenylation signals). Such regulatory sequences are described, for example, in Goeddel; Gene Expression Technology: Methods in Enzymology 185, Academic Press, San Diego, Calif. (1990). Regulatory sequences include those which direct constitutive expression of a nucleotide sequence in many types of host cells and those which direct expression of the nucleotide sequence only in certain host cells (e.g., tissue-specific regulatory sequences). The expression vectors of the invention can be introduced into host cells to thereby produce fusion proteins or peptides, encoded by nucleic acids of the invention.

The recombinant expression vectors of the invention can be designed for expression of the fusion protein in prokaryotic or eukaryotic cells, e.g., bacterial cells such as *E*. *coli,* insect cells (using baculovirus expression vectors), yeast cells or mammalian cells (Goeddel, Gene Expression Technology: Methods in Enzymology 185, Academic Press, San Diego, Calif. (1990)). Alternatively, the recombinant expression vector can be transcribed and translated in vitro, for example using T7 promoter regulatory sequences and T7 polymerase.

Expression of proteins in prokaryotes may be carried out in *E. coli* with vectors containing constitutive or inducible promoters directing the expression of proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson (1988) Gene 67:31-40), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant fusion protein.

Examples of suitable inducible non-fusion *E. coli* expression vectors include pTrc (Amann et al. (1988) Gene 69:301-315) and pET 11d (Studier et al., Gene Expression Technology: Methods in Enzymology 185, Academic Press, San Diego, Calif. (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident lambda prophage harboring a T7 gn1 gene under the transcriptional control of the lacUV5 promoter.

One strategy to maximize recombinant protein expression in *E. coli* is to express the protein in bacteria having an impaired capacity to proteolytically cleave the recombinant protein (Gottesman, Gene Expression Technology: Methods in Enzymology 185, Academic Press, San Diego, Calif. (1990) 119-128). Another strategy is to alter the nucleic acid sequence of the nucleic acid to be inserted into an expression vector so that the individual codons for each amino acid are those preferentially utilized in *E. coli* (Wada et al. (1992) Nucleic Acids Res. 20:2111-2118). Such alteration of nucleic acid sequences of the invention can be carried out by standard DNA synthesis techniques.

In another embodiment, the fusion protein expression vector of the present invention is a yeast expression vector. Examples of vectors for expression in the yeast S. *cerivisae* include pYepSec1 (Baldari et al. (1987) EMBO J. 6:229-234), pMFa (Kurjan and Herskowitz, (1982) Cell 30:933-943), pJRY88 (Schultz et al. (1987) Gene 54:113-123), pYES2 (Invitrogen Corporation, San Diego, Calif.), pGBT9 (Clontech, Palo Alto, Calif.), pGAD10 (Clontech, Palo Alto, Calif.), pYADE4 and pYGAE2 and pYPGE2 (Brunelli and Pall (1993) Yeast 9:1299-1308), pYPGE15 (Brunelli and Pall (1993) Yeast 9:1309-1318), pACT11 (Dr. S. E. Elledge, Baylor College of Medicine), and picZ (InVitrogen Corp, San Diego, Calif.).

Alternatively, fusion proteins of the present invention can be expressed in insect cells using baculovirus expression vectors. Baculovirus vectors available for expression of proteins in cultured insect cells include the pAc series (Smith et al. (1983) Mol. Cell Biol. 3:2156-2165) and the pVL series (Lucklow and Summers (1989) Virology 170:31-39).

In another embodiment, a nucleic acid of the invention is expressed in mammalian cells using a mammalian expression vector. Examples of mammalian expression vectors include pCDM8 (Seed (1987) Nature 329:840), pCl (Promega), and pMT2PC (Kaufman et al. (1987) EMBO J. 6:187-195). When used in mammalian cells, the expression vector's control functions are often provided by viral regulatory elements. For example, commonly used promoters are derived from polyoma virus, Adenovirus 2, cytomegalovirus and Simian Virus 40. For other suitable expression systems for both prokaryotic and eukaryotic cells see chapters 16 and 17 of Sambrook et al. (supra).

In another embodiment, the recombinant mammalian expression vector is capable of directing expression of the nucleic acid preferentially in a particular cell type. Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748).

Another aspect of the invention pertains to host cells into which a recombinant expression vector of the invention or isolated nucleic acid molecule of the invention has been introduced. The term refers not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a fusion protein can be expressed in bacterial cells such as *E. coli*, insect cells, yeast or mammalian cells (such as Chinese hamster ovary cells (CHO) or COS cells).

Vector DNA or an isolated nucleic acid molecule of the invention can be introduced into prokaryotic or eukaryotic cells via conventional transformation or transfection techniques. As used herein, the terms "transformation" and "transfection" are intended to refer to a variety of techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook, *et al.,* and other laboratory manuals.

In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding fusion protein or can be introduced on a separate vector. Cells stably transfected with the introduced nucleic acid can be identified by drug selection.

A host cell of the invention, such as a prokaryotic or eukaryotic host cell in culture, can be used to produce a fusion protein according to the present invention. Accordingly, the invention further provides methods for producing fusion protein using the host cells of the invention. In one embodiment, the method comprises culturing the host cell of the invention (into which a recombinant expression vector or isolated nucleic acid molecule encoding fusion protein has been introduced) in a suitable medium such that fusion protein is produced. In another embodiment, the method further comprises isolating fusion protein from the medium or the host cell.

### Pharmaceutical Compositions

The nucleic acid molecules and polypeptides (also referred to herein as "active compounds") of the invention can be incorporated into pharmaceutical compositions suitable for administration. Such compositions typically comprise the nucleic acid molecule, fusion protein, or antibody and a pharmaceutically acceptable carrier. As used herein the language "pharmaceutically acceptable carrier" includes solvents, dispersion media, coatings, antibacterial and antifungal agents, isotonic and absorption delaying agents, which are compatible with pharmaceutical administration. Additional active compounds may be incorporated into the compositions.

A pharmaceutical composition of the invention is formulated to be compatible with its intended route of administration. Preferable routes of administration include parenteral, e.g., intravenous or intraarterial administration. Solutions or suspensions used for parenteral administration: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions or dispersions and sterile powders for the extemporaneous preparation of sterile injectable solutions or dispersion. For intravenous administration, suitable carriers include physiological saline, Cremophor EL (BASF; Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi.

The carrier can be a solvent or dispersion medium containing, for example, water, ethanol, polyol (for example, glycerol, propylene glycol, and liquid polyetheylene glycol), and mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition.

Sterile injectable solutions can be prepared by incorporating the active compound (e.g., a fusion protein or anti-fusion protein antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

It is especially advantageous to formulate oral or parenteral compositions in dosage unit form for ease of administration and uniformity of dosage. Dosage unit form as used herein refers to physically discrete units suited as unitary dosages for the subject to be treated. Each unit contains a predetermined quantity of active compound calculated to produce the desired therapeutic effect in association with the required pharmaceutical carrier.

The nucleic acid molecules of the invention can be inserted into vectors and used as gene therapy vectors. Gene therapy vectors can be delivered to a subject by, for example, intravenous injection, local administration (US-A 5,328,470) or by stereotactic injection (see, e.g., Chen et al. (1994) Proc. Natl. Acad. Sci. USA 91:3054-3057).

The pharmaceutical preparation of the gene therapy vector may comprise the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

### Uses and Methods of the Invention

The nucleic acid molecules, proteins, protein homologues, and antibodies described herein can be used in one or more of the following methods:
a) methods of treatment (e.g., therapeutic and prophylactic).
b) screening assays;
c) predictive medicine (e.g., diagnostic assays, prognostic assays).

A fusion protein interacts with other cellular proteins, in particular stem cells, and can thus be used for augmenting healing processes directly by differentiation of EPCs into endothelial cells of the vessel wall or indirectly by secretion of positive modulating factors.

The isolated nucleic acid molecules of the invention can be used to express fusion protein (e.g., via a recombinant expression vector in a host cell in gene therapy applications). In addition, the fusion protein can be used to screen drugs or compounds which modulate the fusion protein activity or expression as well as to treat disorders. In addition, the anti-fusion protein antibodies of the invention can be used to modulate fusion protein activity.

### Methods of Treatment

The present invention provides for both preventive and therapeutic methods of treating a subject at risk of (or susceptible to) a cardiovascular disorder or having a cardiovascular disorder associated with exposed subendothelial collagen.

### Preventive Methods

In one aspect, the invention provides a method for preventing in a subject, a disease or condition associated with exposed subendothelial collagen. Subjects at risk for a disease which is caused or contributed to by exposed subendothelial collagen can be identified by, for example, conventional methods for identifying subject at risks of cardiovascular events, such as high LDL cholesterol levels, arterial hypertension, diabetes mellitus, smoking, and by existing and novel biomarkers for instable arterial plaques, such as plaque enhancement in contrast NMR imaging, troponin T and I or RGD peptides.

Specifically, the polypeptide according to the invention is useful for the treatment of cardiovascular disease. Certain cardiovascular disorders are associated with endothelial lesions exposing collagen to platelets. A polypeptide according to the invention can be used to treat such disorders. These disorders include all complications of atherosclerosis, such as acute coronary syndromes (such as myocardial infarctions) and acute or chronic cerebrovascular disorders, such as transient ischemic attacks (TIA) or stroke, cardiac and coronary intervention by percutaneous catheter intervention (PCI) and cardiac surgery,.

Moreover, by pre-incubation of hematopoetic stem cells with the fusion protein and binding of bone marrow collagen via the glycoprotein VI component of the fusion protein, repopulation of the bone marrow by stem cells after transplantation could be improved.

### Therapeutic Methods

The polypeptide of the present invention may be used in a therapeutic method for the prevention or treatment of cardiovascular disease. Preferably, the polypeptide of the present invention is used in the form of a dimer. In particular, the polypeptide of the present invention may be used for homing of progenitor cells to improve vascular repair.

The dosage regimen of the administration of the polypeptide depends on the age, weight, sex, and condition of the subject to be treated. The dosage may preferably be in the range of from 0.01 to 2 g of the polypeptide of the present invention per patient per day. The polypeptide may be administered preferably parenterally. An administration may be 1 to 5 times per day.

In one embodiment, the method involves administering the polypeptide of the present invention in combination with a further agent, or a combination of agents. Examples of further agents are GPVI-Fc, thrombolytic agents such as recombinant tissue plasminogen activator, anti-platelet agents, such as ADP receptor blockers (clopidogrel, ticagrelor, cangrelor, and others), thrombin antagonists (dabigatran or others), or factor X antagonists (such as rivaroxaban), or heparin.

### EXAMPLES

### Material

Besides the kits and materials mentioned in the following method section, the following substances were used. Oligonucleotides were purchased from Eurofins MWG Operon (Ebersberg, Germany). Herculase polymerase (Stratagene, La Jolla, California) was used for PCR amplification. Media for cell cultures and PBS were from Biochrom (Berlin, Germany). Chemicals were from Roth (Karlsruhe, Germany) and Sigma-Aldrich (Seelze, Germany). Bovine collagen I was purchased from BD Biosciences (San Jose, California).

### Amplification and identification of the variable sequences of light and heavy chains of the hybridoma cell line W6B3H10

mRNA was isolated from 4x10⁶ and 1.4x10⁷ cells of the hybridoma cell line W6B3H10 using the Oligotex Direct mRNA kit (QIAGEN, Hilden, Germany) according to the manufacturer's protocol. 18 µl isolated mRNA was taken for cDNA synthesis using the Superscript III Kit (Invitrogen, Carlsbad, California) according to the manufacturer's protocol. To amplify the sequences coding for the variable regions of heavy and light chains of the W6B3H10 antibody, different primer combinations were tested: Bi7/Bi5, Bi8/Bi5 for amplification of kappa light chain variable sequence, Bi3/Bi4, Bi3d/Bi4 for gamma heavy chain variable sequence (primers are described in Dübel S *et al,* 1994). The resulting bands were excised from an agarose gel, purified using the GFX Gel Band Purification Kit (GE Healthcare, Piscataway, New Jersey) and sequenced with Bi5seq (5' GGGAAGATGGATCCAGTTG 3'), Bi5fwd (5' CCATGTCCATGTCACTTG 3'), and Bi5rev (5' GGTTTCTGTTGATACCAG 3') for light chain sequencing. Heavy chain sequences were obtained with the sequencing primers Bi4seq (5' CAGGGGCCAGTGGATAGA 3'), Bi4fwd (5' CTGACCTGATGAAGCCTG 3'), and Bi4rev (5' TTCACCCAGTGCACGTAG 3').

### Expression and purification of single chain antibodies and of fusion proteins

The DNA constructs coding for the single chain antibodies were produced by gene synthesis and cloned (Geneart, Regensburg, Germany) into the mammalian expression vector pcDNA5-FRT (Invitrogen, Carlsbad, California). Transient transfections of CHO cells were done using either Attractene (QIAGEN, Hilden, Germany) or Lipofectamine 2000 transfection reagent (Invitrogen, Carlsbad, California) according to the manufacturers' protocols. Because no expression was detectable with the construct scFv-hl, and expression of scFv-lh in CHO cells was very poor, the DNA of both constructs were subcloned into the bacterial expression vector pET22b(+) (Merck, Darmstadt, Germany) via NcoI/XhoI. The sequences were controlled by sequencing, and E. coli of the expression strain BL21 (DE3) (Merck, Darmstadt, Germany) were transformed using these DNAs. Expression of the single chain antibodies was induced using 0.2 mM IPTG. Isolation of the proteins was performed via Strep-Tactin affinity purification (IBA BioTagnology, Göttingen, Germany) according to the manufacturer's protocol.

The DNA construct coding for scFv-lh-GPVI-FclgG2 in pcDNA5-FRT was ordered from Geneart (Regensburg, Germany). A stably expressing CHO cell line was generated using Lipofectamine 2000 (Invitrogen, Carlsbad California) according to the enclosed protocol. For production of fusion protein at a larger scale, CHO cells were cultivated on T500 triple flasks (NUNC, Rochester, New York). To isolate the fusion protein the cellular supernatant was collected and purified using 1 ml Hi Trap protein G HP columns (GE Healthcare, Piscataway, New Jersey). The isolated protein was dialyzed o/n against PBS.

### Test of binding of the single chain antibodies to CD133 antigen expressed on HEK 293 cells by cellular ELISA

A Poly-L-Lysine 96-well plate (BD Biosciences, San Jose, California) was coated with the CD133 expressing cell line AC133/293 as follows. 1x105 cells in 0.2 ml of medium were added to each well and incubated o/n at 37°C, 5% CO2 to allow cells to attach to the surface of the plate. The next day wells were washed once with 0.2 ml PBS and fixed with 0.1 ml 2% Paraformaldehyde (in PBS, pH 7.4) for 10-20 min at RT. Wells were washed with PBS-T (PBS + 0.1 % Tween-20) and blocked with either 1x RotiBlock or 3% milk in PBS-T for 1 hour at RT. After washing with PBS-T, serial dilutions of the respective antibody were added to the wells and incubated at RT for at least one hour while shaking. After washing with PBS-T, 0.1 ml of StrepMAb-Classic-HRP (1:10000 in PBS-T, Iba BioTagnology, Göttingen, Germany) or of horseradish peroxidase linked anti-mouse IgG (1:10000 in PBS-T, Dianova, Hamburg, Germany) was added and incubated at RT for one hour with shaking. After washing with PBS-T, 0.1 ml of 1-Step Ultra TMB-ELISA substrate (Thermo Scientific, Braunschweig, Germany) was added and incubated until an adequate blue staining developed. To stop the reaction 0.1 ml 1 M H₂SO₄ was added to each well and absorbance at 450 nm and 595 nm as a reference was measured with an infinite F200 plate reader (TECAN, Männedorf, Switzerland). To test for specificity of binding a competitive ELISA was performed with 300 ng/ml (2 nM) W6B3H10 parental mAb and increasing amounts of competitor protein. Signals were detected using an anti-mouse IgG-HRP antibody (1:10000 in PBS-T, Dianova, Hamburg, Germany).

### Binding ELISA with immobilized Collagen I

An Immulon 2 HB 96-well plate (NUNC, Rochester, New York) was coated with 0.1 ml 1 µg/ml bovine collagen I in 15 mM Na2CO3, 35 mM NaHCO3, pH 9.6 o/n at 4°C. Wells were washed with PBS-T, blocked with 0.1 ml 1x RotiBlock (Roth, Karlsruhe, Germany) in PBS-T for one hour and washed again before addition of 0.1 ml of threefold dilutions of fusion protein. After one hour incubation at RT with shaking, wells were washed with PBS-T. Wells were incubated with 0.1 ml antihuman IgG-HRP (Dianova, Hamburg, Germany) 1:10000 in PBS-T for one hour at RT with shaking. The plate was washed with PBS-T and incubated with 0.1 ml 1-Step Ultra TMB-ELISA (PIERCE, Rockford, Illinois). After blue staining had developed sufficiently, reactions were stopped by addition of 0.1 ml 1 M H₂SO₄. Absorbance was measured at 450 nm and 595 nm as a reference wavelength using an infinite F200 plate reader (TECAN, Männedorf, Switzerland). EC₅₀ values were calculated with Sigma Plot 11 using Four Parameter Logistic.

### Adhesion of CD133-expressing cells to Collagen under shear forces

A glass slide was coated with 10 µg/ml collagen I according to Langer et al (2005) and inserted into a flow chamber (Oligene, Berlin, Germany). The collagen coated surface of the slide was pre-treated with 10 µg/ml of the fusion protein for 30 min. To show CD133 dependency of binding, the slide was incubated with W6B3H10 mAb as well. AC133/293 cells were added and incubated under shear forces of 2000 s-1. The experiments were videotaped and evaluated off-line.

### Carotid ligation in mice and assessment of EPC adhesion by intravital microscopy

CD133+ cells were isolated from human cord blood as described (Bueltmann A *et* al, 2003).

To evaluate the effect of the fusion protein on EPC recruitment in vivo, we used intravital fluorescence microscopy as described (Massberg *et al,* 2002). Prior to the experiments, EPCs were stained with 5-carboxyfluorescein diacetate succinimidyl ester (DCF) and incubated with the fusion protein (20 µg/ml/100 nM) or GPVI-Fc (15 µg/ml/100 nM) for 30 min. Wild-type C57BL/6J mice (Charles River Laboratories) were anesthesized by intraperitoneal injection of a solution of midazolame (5 mg/kg body weight; Ratiopharm), medetomidine (0.5 mg/kg body weight; Pfizer) and fentanyl (0.05 mg/kg body weight, CuraMed/Pharam GmbH). Polyethylene catheters (Portex) were implanted into the right jugular vein and fluorescent EPCs (5x104/250µl) were injected intravenously. The common carotid artery was dissected free and ligated vigorously for 5 min to induce vascular injury. Before and after vascular injury, interaction of the fluorescent EPCs with the injured vessel wall was visualized by in situ in vivo video microscopy of the common carotid artery using a Zeiss Axiotech microscope (20x water immersion objective, W 20x/0.5; Carl Zeiss Microlmaging, Inc.) with a 100-W HBO mercury lamp for epi-illumination. The number of adherent EPCs was assessed by counting the cells that did not move or detach from the endothelial surface within 15 s. Their number is given as cells/ mm² endothelial surface.

### RESULTS AND DISCUSSION

### Identification of variable sequences coding for the monoclonal antibody W6B3H10 and construct design

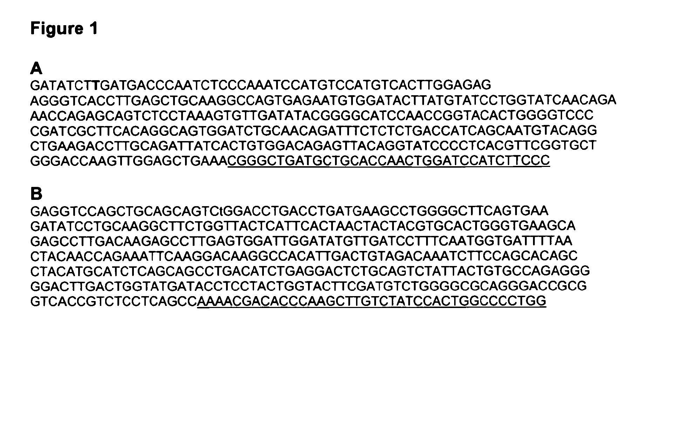

After PCR amplification, which yielded products with each primer combination, bands were excised from the gel, purified and sequenced. The sequences derived from sequencing with three different primers each were assembled (Figure 1) and compared to the IgG data base using IGBlast at NCBI (http://www.ncbi.nlm.nih.gov/igblast/).

To establish the sequences for single chain antibodies (scFv) in two possible orientations "light-heavy" or "heavy-light", a leader sequence each was chosen randomly from the data base V-Base (see http://vbase.mrc-cpe.cam.ac.uk) to facilitate secretion of the antibody into the medium of mammalian cell cultures.

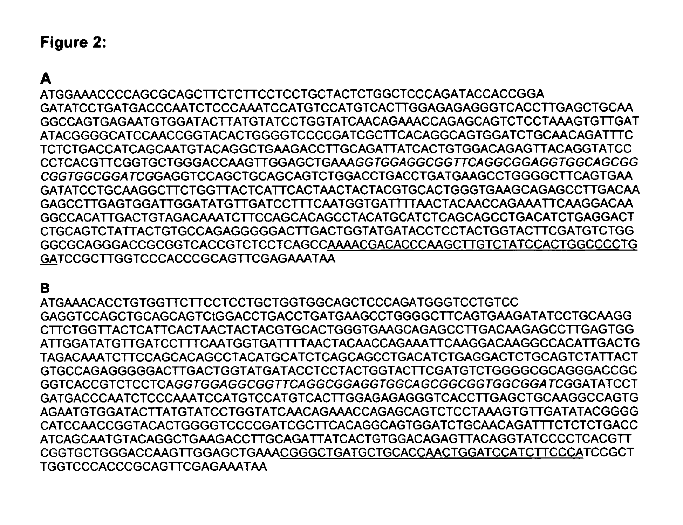

The sequences of heavy and light chains were connected by a Gly-Ser linker coding for Gly Gly Gly Gly Ser Gly Gly Gly Gly Ser Gly Gly Gly Gly Ser. To allow for isolation of the single chain antibodies by affinity purification, a sequence coding for a Strep Tag II was added at the C-terminal end. The established sequences of the constructs named scFv-lh and scFv-hl are shown in Figure 2.

### Expression of the single chain antibodies and test of binding to CD133

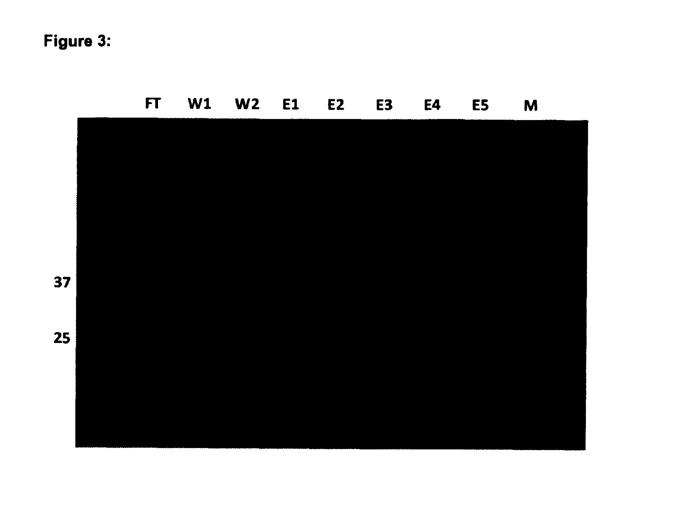

After transient transfection of CHO cells with the single chain constructs, a weak expression could be detected with scFv-lh (see Figure 3).

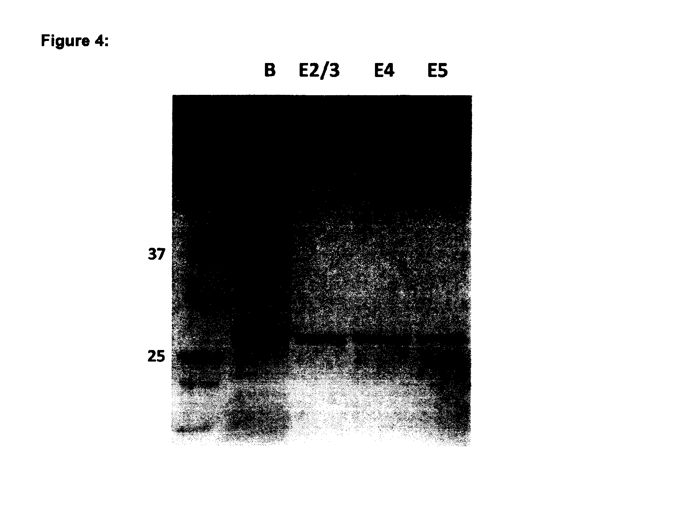

After large scale transfection enough protein could be isolated to be tested for binding to CD133 in a cellular ELISA. In parallel the protein was also expressed in *E.coli* for sufficient stock production. ScFv-hl, which could not be expressed in CHO cells, was produced and purified solely from *E. coli*. (see Figure 4).

To test whether the single chain antibodies recognize their antigen CD133, threefold dilution series of the single chain antibodies were incubated on fixed AC133/293 cells, expressing the CD133 antigen on their surface. Whereas there was a clear binding of scFv-lh with high affinity in the low nanomolar range, surprisingly, affinity of scFv-hl to CD133 (see Figure 5A) was not detected.

This finding was not due to the different expression systems, because scFv-lh purified from *E. coli* showed the same binding characteristics as the one isolated from mammalian cell culture.

To test for specificity of the interaction of scFv-lh with CD133, binding of 2 nM of W6B3H10 mAb to CD133 on fixed AC133/293 cells was competed with increasing amounts of the single chain antibody. This demonstrated clearly that scFv-lh could efficiently block binding of the monoclonal antibody to the antigen CD133 (Figure 5B) with an IC50 value of 3.1 nM, This unexpectedly high affinity might be due to the much smaller size of the molecule, which makes immobilized CD133 for scFv-lh more accessible than for the larger mAb.

### Design, expression and characterization of the fusion protein

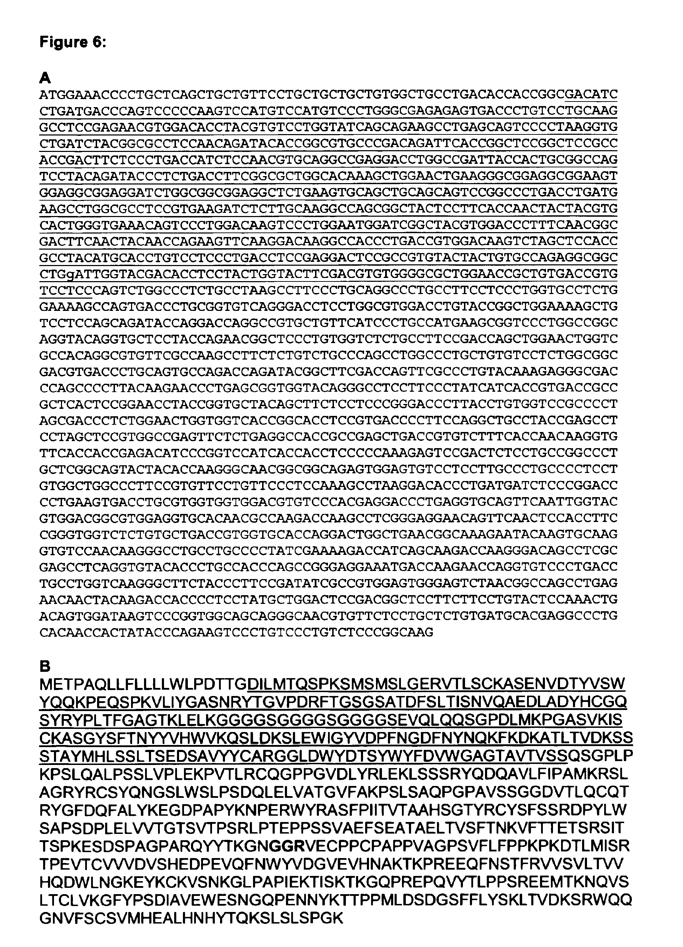

After identification of scFv-lh as constituent for the fusion protein, GPVI-FclgG2 was chosen as second moiety for the bifunctional protein. While GPVI should mediate binding to collagen, the human Fc portion of IgG2 was selected to facilitate affinity purification on the one hand and avoid undesirable effector functions associated with the more commonly used FclgG1 on the other hand. Therefore, the fusion protein was designed in such a manner that the single chain antibody component scFv-lh is followed by soluble glycoprotein VI and FclgG2, which were separated by a three amino acid GGR-linker for more flexibility (Figure 6).

The fusion protein was expressed in adhesion culture of stably transfected CHO cells on T500 triple flasks. Supernatants were purified using Protein G affinity chromatography. A typical yield of the fusion protein was in the range of 2 -2.7 mg/l.

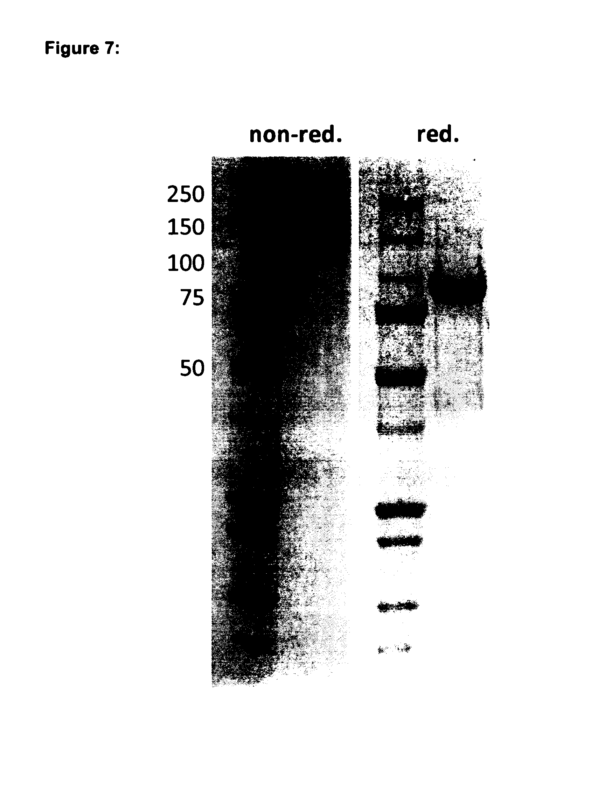

Identity and purity of the protein were controlled by Western blot using an antihuman IgG antibody, linked to horse radish peroxidase (data not shown) and by Coomassie Brilliant Blue stained polyacrylamide gel (Figure 7).

Separation of the fusion protein under reducing conditions resulted in a band size of about 90 kD. The molecular mass under non-reducing conditions was app. 180 kD which showed clearly that the protein exists as dimer.

The theoretical molecular mass of the monomeric protein of 79.1 kD shows that it must be post-translationally modified, most probably by glycosylation.

For platelet glycoprotein VI only one N-linked glycosylation site is described at amino acid 92 (Kunicki et al, 2005). The single chain antibody moiety has no consensus sequence for N-linked glycosylation, whereas the Fc-portion of IgG2 also harbors one N-linked glycosylation site. Because these two glycosylation sites are not sufficient to account for the observed size difference, the fusion protein may contain additional O-linked glycosylation sites.

### Binding characteristics of the fusion protein to CD133 and collagen

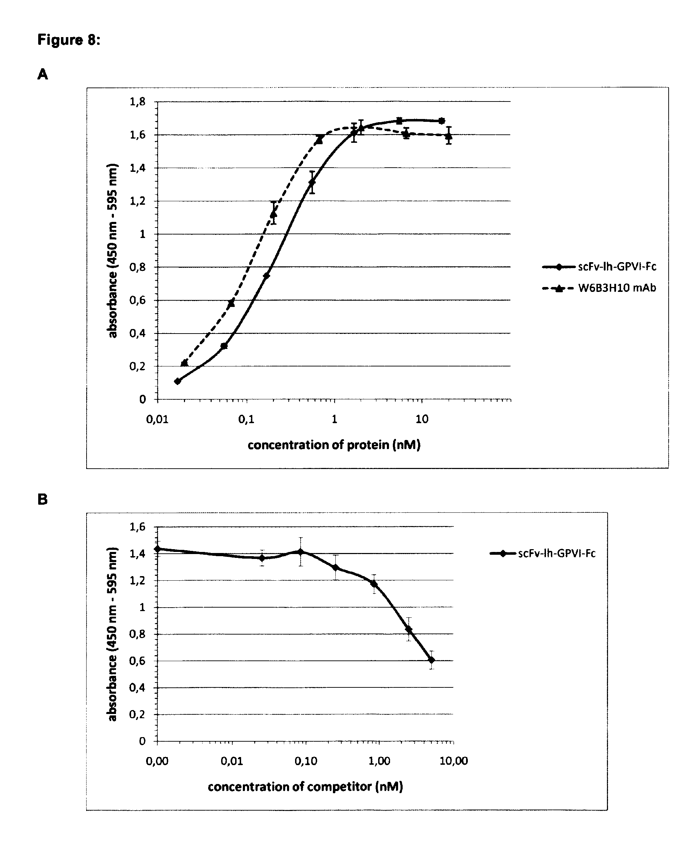

Binding of the fusion protein and of W6B3H10 mAb to CD133 was compared by ELISA with fixed AC133/293 cells (Figure 8A). Observed EC50 values of 0.21 nM for the fusion protein and of 0.12 nM for W6B3H10 mAb were of the same order of magnitude. Thus the binding properties of the fusion protein to the antigen CD133 have improved compared to the single chain antibody, which could only be explained in part by dimerization of the fusion protein, which leads to two identical antigen binding sites similar to the situation found in a full size antibody.

The GPVI-FclgG2 polypeptide subsequent to the single chain polypeptide may have some stabilizing effect on the three dimensional structure of the single chain antibody moiety, which seems to be beneficial for antigen binding. To confirm specificity of binding, a competitive ELISA was performed with 2 nM W6B3H10 mAb and increasing amounts of the fusion protein. Efficient inhibition of binding of the mAb to fixed AC133/293 cells confirmed that binding is mediated by CD133 (see Figure 8B).

Binding of the fusion protein to its second binding partner collagen I was also shown. In an ELISA with immobilized collagen I binding of the fusion protein was compared with that of GPVI-FclgG1. For both a dose dependent binding could be observed, but surprisingly, binding affinities differed (Figure 9A). Specificity of binding of the fusion protein to collagen was confirmed by competitive ELISA with soluble collagen I (Figure 9B).

### Bispecific binding of the fusion protein under dynamic conditions

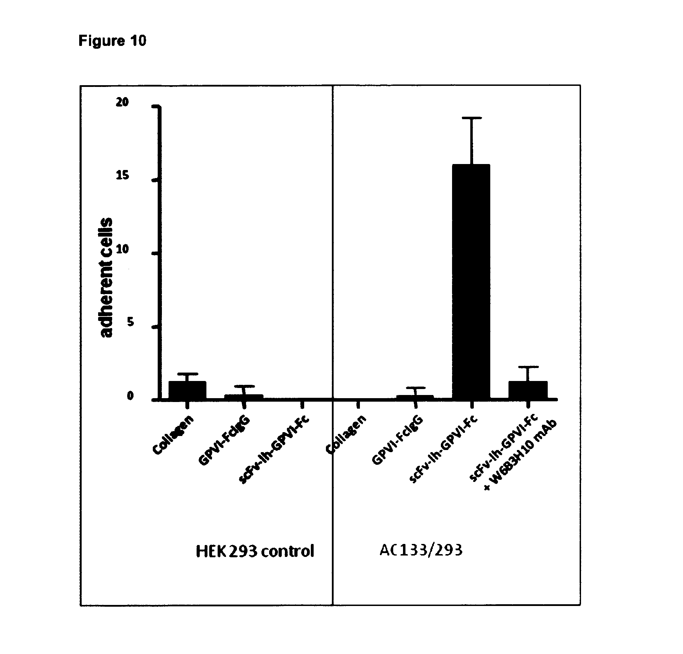

In the previous experiments, binding of the fusion protein to each of its binding partners was demonstrated separately. To confirm the bifunctionality of the molecule by simultaneous binding to both target proteins, a glass slide was coated with 10 µg/ml collagen I, inserted into a flow chamber, pre-incubated with 10 µg/ml of the fusion protein and incubated with CD133 expressing AC133/293 cells under shear forces of 2000/s, mimicking the conditions present in the human blood stream. This experiment demonstrates that the fusion protein very efficiently mediates binding of CD133 expressing cells to collagen even under shear forces. The binding could be reversed by addition of the parental W6B3H10 mAb which shows that immobilization of AC133/293 cells on the collagen surface is CD133-dependent (Figure 10).

### In vivo binding of EPCs to injured blood vessels

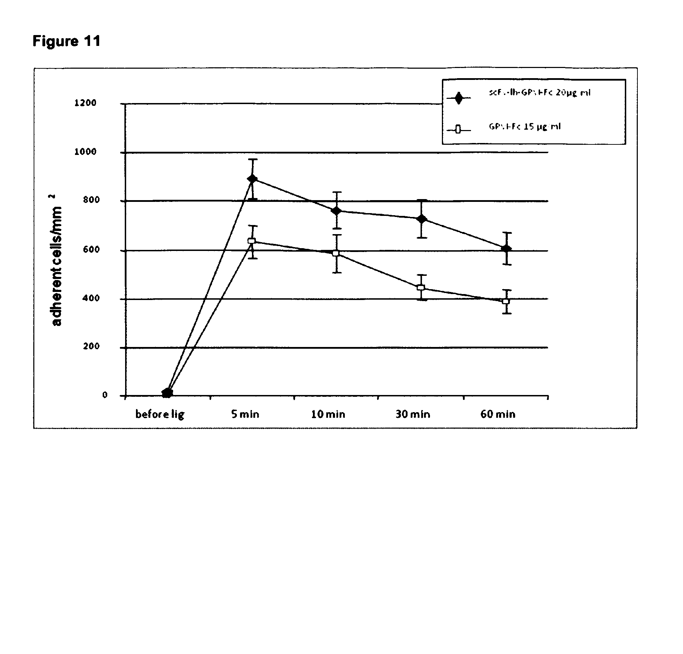

To test whether CD133 expressing EPCs could be efficiently recruited and attached to the wall of an injured blood vessel, EPCs were isolated from human cord blood, fluorescently labeled, and pre-incubated with either the fusion or the control protein GPVI-Fc, which were then applied intravenously into the jugular vein of an anesthesized mouse. After injury of the vessel wall of the carotid artery, the number of attached EPCs was counted at increasing time intervals. It was clearly shown that the fusion protein significantly increased the number of attached EPCs compared to the control protein, with the highest number of cells 5 min after injury, but still significant numbers of cells after 60 min (Figure 11).

The observation that pre-incubation of EPCs with the control protein also led to considerable amounts of adherent EPCs can be explained by the established findings that EPCs adhere to sites of vascular injury which is mediated by platelets (Abou-Saleh et al, 2009). Pre-incubation with the fusion protein could be especially beneficial in patients with risk factors for coronary artery disease, where the number of circulating EPCs is low and who could be treated either by allogeneic EPC transplantation or by ex vivo expanded autologous EPC transplants.

### LITERATURE

Abou-Saleh H, Yacoub D, Théorêt JF, Gillis MA, Neagoe PE, Labarthe B, Théroux P, Sirois MG, Tabrizian M, Thorin E, Merhi Y, Endothelial progenitor cells bind and inhibit platelet function and thrombus formation, Circulation 120 (2009), 2230-2239 Bueltmann A, Gawaz M, Münch G, Ungerer M, Massberg S, Immunoadhesin comprising a glycoprotein VI domain, WO03104282 (2003)
Dübel S, Breitling F, Fuchs P, Zewe M, Gotter S, Welschof M, Moldenhauer G, Little MJ, Isolation of IgG antibody Fv-DNA from various mouse and rat hybridoma cell lines using the polymerase chain reaction with a simple set of primers. Immunol Methods 175 (1994), 89-95.
Kunicki TJ, Cheli Y, Moroi M, Furihata K, The influence of N-linked glycosylation on the function of platelet glycoprotein VI, Blood 106 (2005), 2744-2749
Langer H, May AE, Bültmann A, Gawaz M, ADAM 15 is an adhesion receptor for platelet GPIIb-IIIa and induces platelet activation, Thromb Haemost. 94, (2005), 555-61
Massberg S, Brand K, Grüner S, Page S, Müller E, Müller I, Bergmeier W, Richter T, Lorenz M, Konrad I, Nieswandt B, Gawaz M, A critical role of platelet adhesion in the initiation of atherosclerotic lesion formation, J. Exp. Med. 196 (2002), 887-896 Moroi M & Jung SM, Platelet glycoprotein VI: its structure and function, Thromb Res. 114, (2004), 221-33.

## Claims

1. An isolated nucleic acid molecule selected from the group consisting of:
i. a nucleic acid molecule comprising a nucleotide sequence which is at least 85% identical to the nucleotide sequence of SEQ ID NO:1 or a complement thereof;
ii. a nucleic acid molecule comprising a fragment of at least 1500 consecutive nucleotides of the nucleotide sequence of SEQ ID NO:1, or a complement thereof;
iii. a nucleic acid molecule which encodes a polypeptide comprising an amino acid sequence at least 85% identical to SEQ ID NO:2;
iv. a nucleic acid molecule which encodes a fragment of a polypeptide comprising the amino acid sequence of SEQ ID NO:2, wherein the fragment comprises at least 500 contiguous amino acids of SEQ ID NO: 2; and
v. a nucleic acid molecule which encodes a variant of a polypeptide comprising the amino acid sequence of SEQ ID NO: 2, wherein the nucleic acid molecule hybridizes to a nucleic acid molecule comprising the entire SEQ ID NO: 1, or complement thereof under conditions of incubation at 45 °C in 6.0xSSC followed by washing in 0.2xSSC/0.1% SDS at 65 °C.

2. A nucleic acid molecule according to claim 1, which is selected from the group consisting of:
a) a nucleic acid comprising the nucleotide sequence of SEQ ID NO:1; and
b) a nucleic acid molecule which encodes a polypeptide comprising the amino acid sequence of SEQ ID NO:2
c) a nucleic acid molecule encoding a polypeptide as defined by claim 1 containing a humanized immunoglobulin or parts of an immunoglobulin having binding specificity for CD133

3. The nucleic acid molecule of claim 1 or 2, further comprising vector nucleic acid sequences.

4. A host cell which contains the nucleic acid molecule of any one of claims 1 to 3.

5. A polypeptide capable of simultaneously and selectively binding to collagen and CD133 protein, which is selected from the group consisting of:
a) a fragment of a polypeptide comprising the amino acid sequence of SEQ ID NO: 2, wherein the fragment comprises at least 600 contiguous amino acids of SEQ ID NO: 2;
b) a variant of a polypeptide comprising the amino acid sequence of SEQ ID NO: 2, wherein the variant is encoded by a nucleic acid molecule which hybridizes to a nucleic acid molecule comprising the entire SEQ ID NO: 1 or a complement thereof under conditions of incubation at 45 °C in 6.0xSSC followed by washing in 0.2xSSC/0.1% SDS at 65 °C.;
c) a polypeptide which is encoded by a nucleic acid molecule comprising a nucleotide sequence which is at least 85% identical to a nucleic acid consisting of the nucleotide sequence of SEQ ID NO:1 or the complement thereof; and
d) a polypeptide comprising an amino acid sequence that is at least 85% identical to SEQ ID NO:2.

6. A polypeptide according to claim 5, which comprises the amino acid sequence of SEQ ID NO: 2.

7. A polypeptide as defined by claim 5 or 6 containing a variable region of a humanized immunoglobulin having binding specificity for CD133.

8. The polypeptide according to any one of claims 5 to 7, which is a dimer.

9. A method for producing a polypeptide comprising the amino acid sequence of SEQ ID NO: 2, the method comprising culturing the host cell of claim 4 under conditions in which the nucleic acid molecule is expressed.

10. Pharmaceutical composition comprising the polypeptide according to any one of claims 5 to 8.

11. Polypeptide according to any one of claims 5 to 8 for use in the prevention or treatment or diagnosis of cardiovascular disease.

12. Use of a polypeptide according to any one of claims 5 to 8 for the manufacture of a medicament for the prevention or treatment of cardiovascular disease.

13. Use of a polypeptide according to any one of claims 5 to 8 for preparing a diagnostic marker for unstable plaques.
